# EUROPEAN PATENT SPECIFICATION

(11) **EP 1 053 726 B1**
(45) Date of publication and mention of the grant of the patent: **02.07.2003**
(21) Application number: 00201419.9
(22) Date of filing: 20.04.2000
(51) Int. Cl.: A61F 7/02

(54) **Thermal pack incorporating a temperature indicator**
Thermische Packung mit Temperaturindikator
Pochette thermique comprenant un indicateur de température

(30) Priority: 19.05.1999 GB 9911588
(43) Date of publication of application: 22.11.2000
(73) Proprietor: 3M Innovative Properties Company, St. Paul, Minnesota 55133-3427 (US)
(72) Inventor: SCHATEN, Bernd B., 46322 Borken (DE)
(74) Representative: Aleandri-Hachgenei, Lorraine E.

(56) References cited:
- DE-U- 9 100 061
- US-A- 4 404 820
- US-A- 5 817 149

## Description

The present invention relates to thermal packs which are used to transfer heat to or from an object (typically a part of the human body) and, more especially (but not exclusively), to so-called cold/hot packs which, at the option of the user, can be employed at either a reduced or an elevated temperature. The invention relates in particular to thermal packs of the type comprising a flexible pouch containing a fluid/gel-like material.

Thermal packs which are used only to apply heat to, or only to remove heat from, a body are well known, the simplest forms being hot water bottles and ice packs. Examples of other forms of thermal packs are described in US-A-3 175 558; 3 763 622; 3 893 834; and 4 953 550. Various forms of cold/hot packs are also known. Some cold/hot packs, such as those described in US-A-3 885 403 and 4 756 311, can either be cooled or heated (for example by placing the pack in a freezer or in hot water or a microwave oven) depending on whether it is required to remove heat from or apply heat to an object. Other thermal packs, such as those described in US-A-3 874 504 and 4 462 224, comprise separate compartments of materials which can be mixed to enable the pack to be used as an instant hot pack: in some cases, the pack can be reused, either as a cold pack or as a hot pack, depending on the contents.

It is frequently important to know the temperature of a thermal pack, especially when it is used to transfer heat to/from a part of the human body, for example to relieve pain or reduce swelling. In particular, it is important that the pack should not be at a temperature which will cause too much discomfort or, in extreme cases, damage the skin.

Very often, especially in the case of hot packs such as hot water bottles, a subjective test is used to judge if the pack is too hot. However, that is not always reliable. In an attempt to deal with that problem it has been proposed, in GB-A- 2 257 367, to provide a liquid crystal thermometer on the outside surface of a hot water bottle to indicate if the temperature of that surface is within a restricted acceptable range, or is above or below that range. Similarly, in US-A-5 366 491, it has been proposed to provide a liquid crystal temperature indicating strip on the outside surface of a moist heat pack and, in a heat pack available under the trade designation "Sunbeam", a heat guide has been provided also on the outside surface of the pack. See also DE-U-9100061, which discloses a cushion-pack for thermal therapy having a pack cover made of a flexible foil and a pack filling with warm or cold storing fluid, wherein the cushion-pack is provided with a temperature indicator in the form of strip-shaped sticker.

Liquid crystal indicators have also been proposed for use in other circumstances where it is desirable to know if the temperature of the external surface of a package or of the contents of the package falls within a certain restricted range (see, for example: US-A-4 878 588 and GB-A-2 268 691 which describe feeding bottles incorporating liquid crystal temperature indicators, and US-A-5 645 196 which describes a liquid crystal temperature indicator provided on the inner surface of a holder for milk or fruit juice cartons).

In the case of a cold/hot pack which can be employed at either a reduced or an elevated temperature, it is desirable to be able to provide information concerning the temperature of the pack at both ends of its useful range. It is also desirable, in the case of a pack comprising a flexible pouch containing a fluid/gel-like material, that any temperature indicator employed on such a pack should not interfere with the "feel" of the pack i.e. its softness and flexibility, and should be protected so that it is tamper-proof and difficult to remove.

The present invention provides a thermal pack comprising a flexible pouch containing a fluid/gel-like material, for transferring heat from/to an object; wherein at least a portion of the pouch is transparent, and a flexible temperature indicator is secured to the inside surface of the transparent portion so that it is visible therethrough and is in thermal contact with the fluid/gel-like material.

The present invention also provides a thermal pack for transferring heat from/to an object, the pack comprising a flexible pouch containing a fluid/gel-like material which remains relatively soft and flexible when cooled to a temperature at least as low as -10°C and/or which can be heated to a temperature of at least 60°C; wherein at least a portion of the pouch is transparent, and a flexible temperature indicator is secured to the inside surface of the transparent portion so that it is visible therethrough and is in thermal contact with the fluid/gel-like material.

By way of example only, embodiments of the invention will be described with reference to the accompanying drawings, in which:
Fig. 1 is a view from the front side of a cold/hot pack in accordance with the invention, partly broken away;
Fig. 2 is a view of the pack from the reverse side; and
Fig. 3 is a partial cross-section on an enlarged scale through the pack on the line III-III in Fig. 1.

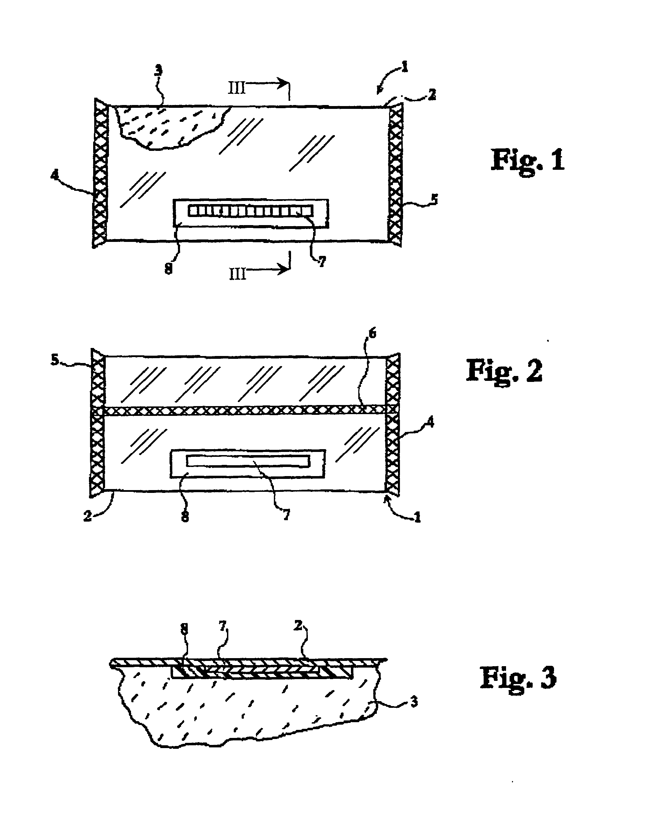

The cold/hot pack 1 shown in the drawings comprises a generally rectangular pouch 2 which is formed from a transparent plastic film material and contains a gel material 3. The pouch 2 is formed from a sheet of film material which is folded and heat-sealed, resulting in seams 4, 5 at each end of the pouch and a central seam 6 extending across the reverse side (Fig. 2).

The cold/hot pack 1 is used to transfer heat to, or from, an object, typically the human body. When used to apply heat to an object, the pack must first be brought to an elevated temperature by, for example, placing it in hot water or in a microwave oven. For applying heat to the human body, the pack would typically be heated to about 60°C and would have the effect of raising the skin temperature of the body (from its normal level of about 30°C) to a level somewhere in the range of from 35 to 45°C for a period of at least 20 minutes. When used to remove heat from an object, the pack must first be brought to a reduced temperature by, for example, placing it in a refrigerator or freezer. For removing heat from the human body, the pack would be cooled to at least -10°C, (typically to about -18°C) and would have the effect of lowering the skin temperature of the body to a level in the range from 10 to 15°C for a period of at least 20 minutes. Packs of that type are known and include, for example, the product available under the trade designation 3M ColdHot Pack™ from Minnesota Mining and Manufacturing Company of St. Paul, Minnesota, USA.

The materials from which the pack 1 is formed are selected to ensure not only that they will withstand repeated heating and cooling of the type described above but also to ensure that the elevated/reduced temperature of the pack is maintained for a reasonable length of time and that the pack remains comparatively flexible and compliant at all temperatures. Suitable plastic film materials for the pouch 2 include polyamide, polyester, polyethylene and polypropylene films and laminates of one or more of those materials. The film material should have a degree of strength appropriate to the intended use of the pack, should be resistant to piercing and should inhibit the transmission of vapour from the gel 3. A preferred film material for a pack which is intended to transfer heat to/from the human body is a polyamide/polyethylene laminate, used with the polyamide layer on the outer side. Suitable gel materials for use inside the pouch 2 are water-based cellulosic gels.

To provide the user of the pack 1 with information about its temperature and reduce the risk of a pack which is too hot or too cold being placed on the skin, the pack is provided with a temperature indicator 7. The indicator 7 is a liquid crystal indicator in the form of a flexible strip and preferably has a range that encompasses the whole temperature range over which the pack 1 is to be used: in the case of a pack 1 which is intended to be used to transfer heat to or from the human body, the indicator encompasses the range -18°C to 60°C and is preferably divided, along its length, into intervals each representing 2°C. The indicator may, for example, be a thermochromic indicator utilising encapsulated liquid crystals, of a type available from International Products & Services srl of Milano, Italy or a thermochromic liquid crystal indicator of a type available from Thermochromic liquid Crystals of Clwyd, United Kingdom. Generally, the temperature indication provided by the indicator 7 can be in the form of numbers, colours, words, symbols, etc..

The indicator strip 7 is secured on the inside of the front side of the pouch 2 so that the temperature that it registers can be seen through the transparent pouch material. The strip 7 may be secured to the pouch material by welding and/or an adhesive and, to protect it from the gel material in the pouch, is advantageously covered with a protective film 8 which may be secured in place by welding and/or an adhesive. In the particular case described above, in which the pouch 2 is formed from a polyamide/polyethylene laminate and the gel material 3 is a water-based cellulosic gel, the indicator strip 7 may be secured in place by a polyacrylic adhesive, and the protective film 8 may be a polyethylene or polypropylene adhesive tape which, after it has been positioned over the indicator strip, is ultrasonically-or laser-welded around its periphery to the pouch material. Generally, the indicator strip 7 (and the protective film 8, when present) should have a degree of flexibility compatible with the material from which the pouch 2 is formed and should be secured in place in a manner that does not unduly detract from that flexibility.

The positioning of the indicator strip 7 on the inside surface of the pouch is facilitated by the construction of the latter from a sheet of film material. The strip 7 is secured in position, and covered with the protective film 8, before any of the seams 4, 5, 6 defining the pouch are formed. When the pack 1 is completed, the indicator strip is completely protected on both sides and cannot be removed but is, nevertheless, clearly visible to the user who can ensure that the pack can safely be applied to the human skin.

In some cases, the indicator strip 7 may be provided with a comparatively wide margin to permit it to be welded to the pouch material.

Although the temperature indicator 7 has been described above as being a single strip, it could instead be made up from two or more strips each encompassing a respective part of the temperature range in which the pack 1 is to be used. It is also not essential for the whole of the pouch 2 of the pack to be formed from a transparent material. The pouch could, for example, be formed from any suitable flexible material provided with a transparent window in the front side where the indicator strip is secured.

Other forms of flexible temperature indicators could be used as an alternative to the liquid crystal indicator 7 described above. The temperature indicator could, for example, be a thermochromic chemical indicator that undergoes a reversible colour change in the temperature range of interest.

It will also be appreciated that, although the temperature indicator strip 7 is described above as being used in a cold/hot pack, it could be used in the same manner in any other type of thermal pack comprising a flexible pouch incorporating a fluid/gel-like material.

## Claims

1. A thermal pack (1) comprising a flexible pouch (2) containing a fluid/gel-like material (3) for transferring heat from/to an object and a temperature indicator, **characterized in that** the temperature indicator (7) is flexible and at least a portion of the pouch is transparent, and the flexible temperature indicator (7) is secured to the inside surface of the transparent portion so that it is visible therethrough and is in thermal contact with the fluid/gel-like material.

2. A pack as claimed in claim 1, in which the fluid/gel-like material (3) remains relatively soft and flexible when cooled to a temperature at least as low as -10°C and/or which can be heated to a temperature of at least 60°C.

3. A pack as claimed in claim 1 or claim 2, in which the temperature indicator (7) is a liquid crystal temperature indicator.

4. A pack as claimed in any one of the preceding claims, in which the temperature indicator is in the form of a flexible strip which is secured by welding and/or an adhesive to the inside surface of the transparent portion of the pouch.

5. A pack as claimed in claim 4, in which the flexible strip has an outer margin of a plastic film material by which the strip is secured to the pouch.

6. A pack as claimed in claim 4 or claim 5, including a layer of a protective material (8) secured over the flexible strip on the inside surface of the transparent portion of the pouch.

7. A pack as claimed in claim 6, in which the protective layer is secured over the flexible strip by welding and/or an adhesive.

8. A pack as claimed in claim 6 or claim 7, in which the protective layer is a plastic film material.

9. A pack as claimed in any one of the preceding claims, in which the pouch has a seam (6) extending across one face, and the temperature indicator is secured on the inside surface of the opposite face.

10. A pack as claimed in any one of the preceding claims, in which the temperature indicator is operable to indicate temperatures over at least the range of from -18°C to 60°C.

11. A pack as claimed in any one of the preceding claims, in which the pouch is formed from a transparent plastic film material.

## Patentansprüche

1. Thermopackung (1) einen flexiblen Beutel (2) umfassend, der ein fluid-/gelartiges Material (3) enthält, zur Übertragung von Wärme von einem/an ein Objekt, und einem Temperaturindikator, **dadurch gekennzeichnet, dass** der Temperaturindikator (7) flexible ist, und dass mindestens ein Abschnitt des Beutels transparent ist und der flexible Temperaturindikator (7) so an der Innenfläche des transparenten Abschnitts befestigt ist, dass er durch diesen hindurch sichtbar ist und im thermischen Kontakt mit dem fluid-/gelartigen Material steht.

2. Packung nach Anspruch 1, in der das fluidgelartige Material (3) relativ weich und flexibel bleibt, wenn es auf eine Temperatur von mindestens -10°C gekühlt wird und/oder auf eine Temperatur von mindestens 60°C erhitzt werden kann.

3. Packung nach Anspruch 1 oder 2, bei der der Temperaturindikator (7) ein Flüssigkristalltemperaturindikator ist.

4. Packung nach einem der vorhergehenden Ansprüche, bei der der Temperaturindikator die Form eines flexiblen Streifens hat, der durch Schweißen und/oder einen Klebstoff an der Innenfläche des transparenten Abschnitts des Beutels befestigt ist.

5. Packung nach Anspruch 4, bei der der flexible Streifen einen Außenrand aus einem Kunststofffolienmaterial hat, mit dem der Streifen an dem Beutel befestigt ist.

6. Packung nach Anspruch 4 oder 5, die eine Schicht aus einem Schutzmaterial (8) umfasst, die über dem flexiblen Streifen auf der Innenfläche des transparenten Abschnitts des Beutels befestigt ist.

7. Packung nach Anspruch 6, bei der die Schutzschicht durch Schweißen und/oder einen Klebstoff über dem flexiblen Streifen befestigt ist.

8. Packung nach Anspruch 6 oder 7, bei der die Schutzschicht ein Kunststofffolienmaterial ist.

9. Packung nach einem der vorhergehenden Ansprüche, bei der der Beutel eine sich über eine Seite erstreckende Naht (6) aufweist und der Temperaturindikator an der Innenfläche der gegenüberliegenden Seite befestigt ist.

10. Packung nach einem der vorhergehenden Ansprüche, bei der der Temperaturindikator dazu dient, Temperaturen in mindestens dem Bereich zwischen -18°C und 60°C anzuzeigen.

11. Packung nach einem der vorhergehenden Ansprüche, bei der der Beutel aus einem transparenten Kunststofffolienmaterial gebildet ist.

## Revendications

1. Pochette thermique (1) comprenant une vessie souple (2) contenant une matière de type fluide/gel (3), en vue d'assurer un transfert de chaleur depuis/vers un objet, et un indicateur de température, **caractérisée en ce que** 1' indicateur (7) est flexible et qu'une partie au moins de la vessie est transparente, et l'indicateur de température souple (7) est fixé sur la surface intérieure de la partie transparente de façon à être visible à travers elle et à être au contact thermique de la matière de type fluide/gel.

2. Pochette selon la revendication 1, dans laquelle la matière de type fluide/gel (3) reste relativement molle et souple lorsqu'elle est refroidie à une température descendant au moins jusqu'à -10°C et/ou susceptible d'être chauffée jusqu'à une température d'au moins 60°C.

3. Pochette selon la revendication 1 ou la revendication 2, dans laquelle l'indicateur de température (7) est un indicateur de température à cristaux liquides.

4. Pochette selon l'une quelconque des revendications précédentes, dans laquelle l'indicateur de température prend la forme d'une bande souple fixée par soudage et/ou adhésif sur la surface intérieure de la partie transparente de la vessie.

5. Pochette selon la revendication 4, dans laquelle la bande souple possède une marge extérieure constituée d'une matière de film plastique par laquelle la bande est fixée sur la vessie.

6. Pochette selon la revendication 4 ou la revendication 5, comportant une couche de matière protectrice (8) fixée par-dessus la bande souple sur la surface intérieure de la partie transparente de la vessie.

7. Pochette selon la revendication 6, dans laquelle la couche protectrice est fixée par-dessus la bande souple par soudage et/ou adhésif.

8. Pochette selon la revendication 6 ou la revendication 7, dans laquelle la couche protectrice est une matière de film plastique.

9. Pochette selon l'une quelconque des revendications précédentes, dans laquelle la vessie présente un joint (6) se prolongeant en travers d'une face, et l'indicateur de température est fixé sur la surface intérieure de la face opposée.

10. Pochette selon l'une quelconque des revendications précédentes, dans laquelle l'indicateur de température peut être mis en oeuvre pour indiquer des températures appartenant au moins à l'intervalle allant de -18°C à 60°C.

11. Pochette selon l'une quelconque des revendications précédentes, dans laquelle la vessie est formée d'une matière de film plastique transparente.
